Europäisches Patentamt

European Patent Office

Office européen des brevets

(19)

(11) Veröffentlichungsnummer: **0 220 427**
**B1**

(12) **EUROPÄISCHE PATENTSCHRIFT**

(45) Veröffentlichungstag der Patentschrift:
**13.12.89**

(21) Anmeldenummer: **86112043.4**

(22) Anmeldetag: **01.09.86**

(51) Int. Cl.⁴: **A61F 2/30**

(54) **In einen Röhrenknochen einsetzbare Markraumsperre.**

(30) Priorität: **10.10.85  CH 4373/85**

(43) Veröffentlichungstag der Anmeldung:
**06.05.87 Patentblatt 87/19**

(45) Bekanntmachung des Hinweises auf die Patenterteilung:
**13.12.89 Patentblatt 89/50**

(84) Benannte Vertragsstaaten:
**AT DE FR GB IT**

(56) Entgegenhaltungen:
**EP-A- 0 006 408**
**BE-A- 876 941**
**CH-A- 559 544**
**FR-A- 2 056 934**
**US-A- 4 276 659**

(73) Patentinhaber: **GEBRÜDER SULZER AKTIENGESELLSCHAFT, Zürcherstrasse 9, CH-8401 Winterthur(CH)**
Patentinhaber: **Protek AG, Stadtbachstrasse 64, CH-3012 Bern(CH)**

(72) Erfinder: **Frey, Otto, Wallrütistrasse 65, CH-8400 Winterthur(DE)**
Erfinder: **Koch, Rudolf, Oberdorfstrasse 229, CH-8267 Berlingen(DE)**

(74) Vertreter: **Dipl.-Ing. H. Marsch Dipl.-Ing. K. Sparing Dipl.-Phys.Dr. W.H. Röhl Patentanwälte, Rethelstrasse 123 Postfach 14 02 68, D-4000 Düsseldorf(DE)**

## Beschreibung

Die Erfindung betrifft eine in einen Röhrenknochen einsetzbare Markraumsperre zur Abschirmung des Markhohlraumes gegen Knochenzement, bestehend aus mindestens elastisch verformbaren Blättern.

Um ein Eindringen von Knochenzement in den an die operativ geschaffene Ausnehmung für den Einsatz eines Prothesenschaftes anschliessenden Markhohlraum eines Röhrenknochens zu verhindern, verwendet man sogenannte Markraumsperren oder -stopfen. Eine Markraumsperre der vorstehend genannten Art für sich in Einsetzrichtung verengende Knochen ist aus der EP-A 0 006 408 bekannt; sie besteht aus einem pfropfenartigen, zylindrischen oder konischen Kern, an den in einem Kranz hohlkegelartig angeordnete Blätter angesetzt sind. Die eigentliche, für Knochenzement undurchlässige Markraumsperre besteht hier aus dem massiven Kern, während die Blätter als Verankerungselemente dienen. Die Blätter sind daher flexibel und durch Längsschlitze voneinander getrennt; sie umschliessen zumindest im verformten Zustand kelchartig einen Hohlraum, der zur Operationsöffnung hin geöffnet ist. Kern und Blätter bestehen dabei aus Kunststoff.

Befindet sich das Ende der erwähnten operativen Ausnehmung jedoch in einem sich in Einsetzrichtung erweiternden Knochen, so ist die genannte Konstruktion ungeeignet, da sie nicht mit der notwendigen Sicherheit in einer bestimmten Höhe fixiert werden kann.

Für derartige Fälle ist eine ebenfalls aus Kunststoff gefertigte spreizbare Markraumsperre bekannt, die aus einem elastisch verformbaren Aussenkörper besteht. In diesen wird ein Spreizkörper von der der operativen Ausnehmung abgewandten Seite her eingezogen. Der Spreizkörper spreizt den Aussenkörper auf und fixiert ihn im Knochenhohlraum, wobei er sich selbst mit dem Aussenkörper verzahnt und so gehalten wird (EP-A 0 058 744). Diese Konstruktion ist aufwendig und in ihrer Einsetztechnik kompliziert.

Obwohl die genannten Konstruktionen mit Durchgangsbohrungen versehen sind, besteht bei ihnen das Problem, dass beim Einbringen des Knochenzementes Blut und Fettgewebe, die sich in der Operationsausnehmung in Bodennähe sammeln, nicht in genügendem Masse aus dem mit dem Zement zu füllenden Hohlraum verdrängt werden. Es bilden sich daher in dem Knochenzementbett Einschlüsse von Blut und Fettgewebe und/oder Luft, die zu einer Festigkeitseinbusse des Zementbettes führen.

Aus der FR-A 2 056 934 ist ein aus einem Metallnetz bestehender Einsatz für den Hüftknochen bekannt; durch diesen Einsatz, der in gewissem Masse für Knochenzement durchlässig ist, soll die in den Knochen eindringende Knochenzementmenge dosiert und begrenzt werden.

Der Erfindung liegt die Aufgabe zugrunde, eine einfache und einfach zu handhabende Markraumsperre zu schaffen, die sowohl in sich verengende als auch in sich erweiternde Knochenhohlräume eingesetzt und in diesen fixiert werden kann und die darüberhinaus eine relativ grosse Durchlässigkeit für Blut, Fettgewebe und Gase hat, jedoch für Knochenzement undurchlässig ist.

Erfindungsgemäss wird diese Aufgabe gelöst durch eine mindestens dreiblättrige Rosette aus Metall als für Blut und Fett durchlässige, für pastenförmigen Knochenzement jedoch undurchlässige Sperre, deren blütenkranzartig angeordnete Blätter elastisch und bleibend verformbar sind und sich im eingesetzten Zustand berühren und/oder überlappen.

Die hohe plastische und elastische Verformbarkeit von Metallen – es können für die neue Markraumsperre die in der Implantat-Technik üblichen Metalle oder Metall-Legierungen verwendet werden, insbesondere Titan oder Titan-Legierungen – erlaubt eine ungefähre Anpassung der Markraumsperre an den Operationshohlraum mit Hilfe einer plastischen Verformung, wobei gleichzeitig das die Fixierung bestimmende Mass der zusätzlichen elastischen Verformung eingestellt wird. Eine Porosität – beispielsweise durch eine Netzstruktur oder die Verwendung von gelochten Blechen – macht die Markraumsperre für Gase und niedrig viskose Flüssigkeiten, wie Blut, durchlässig, während der pastenförmige Knochenzement die Sperre nicht durchdringen kann. Beim Einfüllen des Zements werden auf diese Weise Gase, Blut, Fett und Sekrete aus dem Operationshohlraum verdrängt, so dass sich keine Einschlüsse in dem polymerisierenden Knochenzement ergeben, die zu einer Schwächung des Zementbettes führen.

Bei einer besonders einfachen und vorteilhaften Ausführungsform besteht die Rosette aus einem Drahtnetz, das – falls beispielsweise aus Festigkeitsgründen erforderlich – aus mehreren Lagen aufgebaut sein kann.

Für eine gute Fixierung mittels einer elastischen Klemmung hat es sich als zweckmässig erwiesen, wenn die Blätter der Rosette aus ihrer Grundebene zu einem Kelch abgewinkelt sind. Um den umgebenden Knochen vor Verletzungen zu schützen, ist es weiterhin nützlich, wenn die freien Enden der Blätter gegen die Mitte der Rosette umgebogen sind.

Im folgenden wird die Erfindung anhand eines Ausführungsbeispiels im Zusammenhang mit der Zeichnung näher erläutert.

Fig. 1 und 2 zeigen in einer Aufsicht und in einer Seitenansicht eine Ausführungsform der neuen Markraumsperre;

Fig. 3 ist ein Längsschnitt durch einen sich nach "innen" erweiternden Knochenhohlraum, in den die neue Markraumsperre eingesetzt ist.

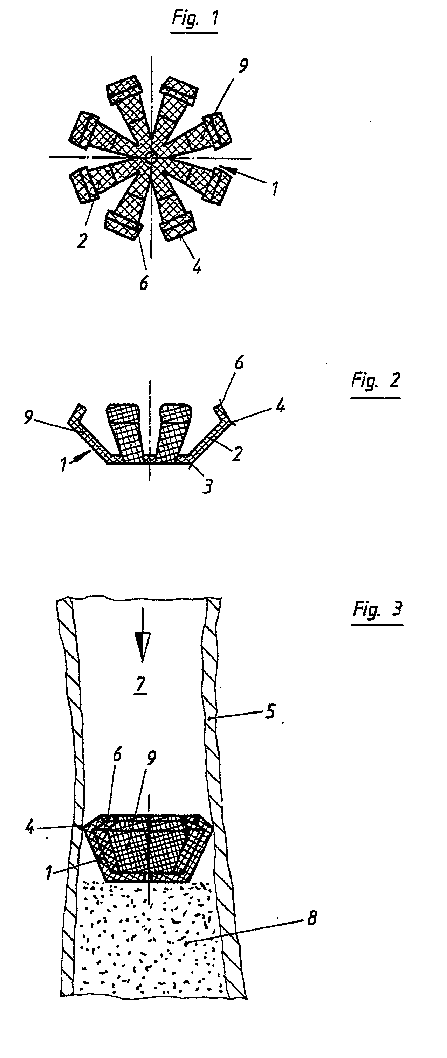

Die in Fig. 1 in einer Aufsicht gezeigte Rosette 1 ist aus einem Drahtnetz aus Titan oder einer Titan-Legierung, beispielsweise durch Stanzen, hergestellt. Sie hat acht einzelne Blätter 2, die in ihrer Breite vom Kern aus nach aussen kontinuierlich zunehmen. Die beim Stanzen zunächst ebenen Blätter 2 des Drahtnetzes 1 sind an den Stellen 3 und 4 bleibend verformt und zu einem Kelch (Fig. 2) abgebogen.

Beim Einsetzen in einen Röhrenknochen 5, das in Fig. 3 von oben her erfolgt ist, erfährt das bleibend zu den Kelchen verformte Drahtnetz 1 in den "Knicken" 3 eine zusätzliche elastische Verformung, die eine Fixierung im Knochen 5 durch Klemmen bewirkt.

Die Knicke an den Stellen 4 haben die Aufgabe, den Knochen 5 beim Einsetzen der Markraumsperre vor Beschädigungen durch die zwar abgerundeten Kanten 6 an den Enden der Blätter 2 zu schützen.

Wie Fig. 3 zeigt, bildet das Drahtnetz 1 nach dem Einsetzen in den Knochen 5 eine "makroskopisch" geschlossene Sperre für den Durchtritt von Knochenzement aus der Operationsöffnung 7 in den mit Mark und Spongiosa 8 gefüllten "unbeschädigten" Knochen. Gleichzeitig ist das Netz 1, dessen für Blut, Fett und Gase durchlässige Porosität durch seine Maschenöffnungen 9 bewirkt wird, eine Trennwand zwischen dem Knochenzement und körpereigenen Flüssigkeiten und Geweben, so dass der Zement nicht verunreinigt und damit in seiner Festigkeit geschwächt wird.

## Patentansprüche

1. In einen Röhrenknochen (5) einsetzbare Markraumsperre (1) zur Abschirmung des Markhohlraumes (8) gegen Knochenzement, bestehend aus mindestens elastisch verformbaren Blättern (2), gekennzeichnet durch eine mindestens dreiblättrige Rosette (1) aus Metall als für Blut und Fett durchlässige, für pastenförmigen Knochenzement jedoch undurchlässige Sperre, deren blütenkranzartig angeordnete Blätter (2) elastisch und bleibend verformbar sind und sich im eingesetzten Zustand berühren und/oder überlappen.

2. Markraumsperre nach Anspruch 1, dadurch gekennzeichnet, dass die Rosette (1) aus einem Drahtnetz besteht.

3. Markraumsperre nach Anspruch 2, dadurch gekennzeichnet, dass die Rosette (1) aus mehreren Lagen aufgebaut ist.

4. Markraumsperre nach Anspruch 1, dadurch gekennzeichnet, dass die Blätter (2) der Rosette (1) aus ihrer Grundebene zu einem Kelch abgewinkelt sind.

5. Markraumsperre nach Anspruch 4, dadurch gekennzeichnet, dass die freien Enden (6) der Blätter (2) gegen die Mitte der Rosette (1) umgebogen sind.

## Claims

1. A marrow chamber barrier (1) introducible into a medullated bone to screen the medullar cavity (8) from bone cement, the barrier comprising at least resiliently deformable strips (2), characterised by an at least three-strip metal rosette (1) as an element pervious to blood and fat but impervious to paste-like bone cement, the strips or leaves or the like (2) of the barrier being arranged like a wreath and being resiliently and permanently deformable and when fitted contacting and/or overlapping one another.

2. A barrier according to claim 1, characterised in that the rosette (1) is made of a wire mesh.

3. A barrier according to claim 2, characterised in that the rosette is devised from a number of layers.

4. A barrier according to claim 1, characterised in that the strips (2) of the rosette (1) are bent from their base plane to form a calyx.

5. A barrier according to claim 4, characterised in that the free ends (6) of the strips (2) are bent round towards the centre of the rosette (1).

## Revendications

1. Bouchon de colmatage (1) composé de lamelles (2) au moins déformables élastiquement, et insérable dans un os tubulaire (5) en vue de protéger la cavité médullaire (8) d'une pénétration d'ostéociment, caractérisé par une rosette (1) en métal comprenant au moins trois lamelles, faisant fonction de bouchon de colmatage perméable au sang et aux graisses, mais imperméable à l'ostéociment pâteux, dont les lamelles (2) disposées à la manière d'une corolle sont déformables élastiquement et durablement, et se touchent et/ou se chevauchent en condition incorporée.

2. Bouchon de colmatage selon la revendication 1, caractérisé par le fait que la rosette (1) consiste en un entrelacs de fils métalliques.

3. Bouchon de colmatage selon la revendication 2, caractérisé par le fait que la rosette (1) est composée de plusieurs couches.

4. Bouchon de colmatage selon la revendication 1, caractérisé par le fait que les lamelles (2) de la rosette (1) sont coudées à l'écart de leur plan de base, pour former un calice.

5. Bouchon de colmatage selon la revendication 4, caractérisé par le fait que les extrémités libres (6) des lamelles (2) sont recourbées vers le centre de la rosette (1).

_Fig. 1_

_Fig. 2_

_Fig. 3_